# EUROPEAN PATENT SPECIFICATION

(11) **EP 0 670 160 B1**
(45) Date of publication and mention of the grant of the patent: **14.07.1999**
(21) Application number: 94203112.1
(22) Date of filing: 26.10.1994
(51) Int. Cl.: A61K 9/46

(54) **Granular product or tablet containing an effervescent system and an active pharmaceutical substance, as well as a method for its preparation**
Ein Brausesystem und einen Arzneiwirkstoff enthaltendes granuläres Produkt bzw. Tablette sowie Verfahren zu deren Herstellung
Produit granulaire ou comprimé contenant un système effervescent et un agent actif pharmaceutique, et son procédé de préparation

(30) Priority: 01.03.1994 DE 4406641; 23.03.1994 CH 87394
(43) Date of publication of application: 06.09.1995
(73) Proprietor: Gergely, Gerhard, Dr., A-1053 Wien (AT)
(72) Inventor: Gergely, Gerhard, Dr., A-1053 Wien (AT); Gergely, Thomas, Dr., A-1053 Wien (AT); Gergely, Irmgard, A-1053 Wien (AT); Gergely, Stefan, Dr., A-1053 Wien (AT)
(74) Representative: Büchel, Kurt F., Dr.

(56) References cited:
- EP-A- 0 415 326
- WO-A-93/00886
- GB-A- 1 270 781
- US-A- 4 704 269

## Description

This invention relates to a granular pharmaceutical preparation or more particularly a tablet containing an effervescent system and a - preferably acid-sensitive - pharmaceutical substance, such as cisapride, beta-carotene, an H2 blocker such as cimetidine or ranitidine, and/or a substance which is to be administered in an effervescent pharmaceutical preparation with comparatively small amounts of effervescent components or a comparatively low acid-binding capacity.

### Background of the Invention

Heretofore it has been possible only with difficulty to incorporate acid-sensitive drugs in stable form into effervescent tablets or effervescent instant granular products, since in contact with the acid of the effervescent system such compositions hydrolyze or decompose, i.e. they are not shelf-stable. Furthermore, whenever such a substance also affects the surface tension of water, frothing occurs which is highly undesirable for the consumption of the effervescent solution, or in any event, hydrophobic particles of the drug tend to creep upward on the glass. On the other hand, in certain cases, the antacid side-effect of an effervescent tablet is undesirable for many drugs. Therefore an object of this invention is to provide an effervescent system which will avoid the aforesaid disadvantages and offer the possibility of administering to a patient pharmaceutical substances, inclusive of acid-sensitive substances which have hydrophobic properties or properties influencing the surface tension of water, in pleasant-to-drink effervescent solutions. It is a further object of this invention to create an effervescent tablet or an instant effervescent granular product with an acid binding capacity of less than 5 meq, in order to avoid undesired antacid effects. This is especially advantageous for all H2 blockers. Lastly, it is desired that the tablet or granular product is to dissolve rapidly in water at a temperature of about 15-20°C in less than about 2 minutes.

### Summary of the Invention

The solution to the aforesaid problems can be achieved in a surprisingly simple, cost-effective and efficient manner in accordance with this invention e.g. by first substantially coating acid particles with a composition comprising at least one neutral substance which causes a depression of the melting point of the acid grains at their surface, and thereafter anchoring thereon at least one second coating which contains an alkali and/or alkaline earth carbonate and/or bicarbonate, and optionally a partial reaction product of the carbonate or bicarbonate with the same or a different organic acid.

The invention is more fully discussed in detail below along with a detailed discussion and illustration of several preferred embodiments.

### Detailed Description

Neutral substances within the meaning of this invention include water soluble polymers, such as e.g. polyvinylpyrrolidone, carbohydrates, such as saccharose, pentaerythritol, glucose, and fructose (although the latter two, under the influence of the only slightly alkaline effervescent-grain surface due to the bicarbonate coating, are subject to a Maillard reaction tending to make them yellow and therefore they are not particulary preferred herein), hydrocolloids, such as maltodextrin, dextrin and the like; especially preferred are higher alcohols, such as xylitol, mannitol and sorbitol.
Various embodiments of the invention are described in the defining clauses of the dependent claims.

It is true that W093/00886 discloses that a foreign acid, possibly gluconic acid delta-lactone, which hydrolyzes to gluconic acid, can be incorporated at the surface of acid vehicle crystals, with the result that the crystal lattice is disturbed and a melting point depression is achieved. However, such a measure cannot of course provide adequate protection for acid-sensitive active substances. It has therefore also been impossible hitherto to use the invention of WO93/00886 for acid-sensitive active substances in practice.

It has also been proposed (British Patent 1,270,781) to coat acid vehicle crystals for effervescent tablets with a thin polymer layer, such as, for example, with polyvinyl-pyrrolidone, carboxymethylcellulose or the like. However, this results in an undesirable retardation of the dissolution time and, in the case of the 1 to 5% by weight of polyvinylpyrrolidone described there in the Examples, foam formation problems; furthermore, some acid is always transferred from the vehicle crystal to the layer in solution when the coating is applied by means of ethanolic or aqueous solution, whereby the acid-sensitive active substances would not be protected sufficiently. In addition, however, those skilled in the art have for over 20 years been unable satisfactorily to solve the problem of accommodating acid-sensitive active substances in effervescent systems not only in a shelf-stable manner but also in relatively small tablet weights with very low acid binding capacity and short dissolution time. An effervescent tablet is generally defined as being particularly rapid when the dissolution (or complete suspending) of the tablet components takes less than 120 sec, preferably 90 sec or less.

According to the invention, however, after (preferably only a small amount of) the neutral substance has been applied to the acid grains, alkali and/or earth alkaline carbonate and/or bicarbonate particles are anchored on the grain surface in order to prohibit an interaction between the acid and the active substance.

Furthermore, the process proposed in EP-A1-415 326 for coating acid vehicle crystals with several times the amount of sugar in order, in combination with bicarbonate, to achieve a slightly prickling effect, for a chewable tablet or lozenge has not been able to solve the combination of the problems or tasks: such a system would not be sufficiently reactive to dissolve an effervescent tablet in water within a reasonable time. It was the aim of the said EP-A1 to slow down the reaction between acid and carbonate in order not to produce an undesired high effervescent effect in the mouth.

If, as disclosed in the prior art (US-A-4 127 645), a tablet having a core of acid, bicarbonate and calcium were coated with a neutral substance, for example with sorbitol solution, such a tablet would not provide reliable protection for acid-sensitive active substances contained in the core. However, if the mixture were pressed with a neutral substance (e.g. maltodextrin, if necessary as a mixture with sugar, US-A-4 650 669; sorbitol with vitamins, US-A-5 223 264, only suitable as a prickling chewable tablet) to give tablets, then either both reactants would be coated together or undesirable agglomerated granules would occur. In both cases, the reaction on dissolution of the tablet would take place too slowly and the dissolution time would thus be undesirably increased, or the solution would contain undesirably large amounts of sugar. Furthermore, it is very probable that, in agglomerated granules, acid particles too would be present unprotected at the surface of the granules; however, this results in greater instability for acid-sensitive active substances.

In U.S. Patent No. 4,867,942, a method is described in which vehicle crystals of a solid, edible organic acid are covered on their surface with a pre-reacted solution serving as buffer, particularly an acid alkali and/or alkaline earth salt of a solid, edible organic acid. Thereafter, more of the acid crystals and amounts of carbonate or bicarbonate are anchored side by side on this coating. Water which is released in the various neutralization partial reactions is removed by a final treatment with alcohol and vacuum drying. Such a process is disadvantageous, however, in that, for acid-sensitive drugs, on the acid crystal surface an additional acid simultaneously enters into a reaction with the alkali carbonate, and the reaction thus proceeds too fast and consequently not sufficiently uniformly. Therefore, the product that forms from this method cannot completely prevent the reaction of an acid-sensitive drug mixed in with it, due to the acid crystals lying on the surface of the granules.

In contrast, the structure of the effervescent system according to this invention not only prevents direct contact of an acid-sensitive drug with the acid crystals thereby providing an effervescent tablet or granular product with substantially more shelf-stability, but it also permits the preparation of substantially smaller tablets, i.e., those with smaller amounts of effervescent components which, when dissolved, result in a buffer system. Thus, the present tablets according to the invention, in contrast to buffer systems of antacid effervescent preparations, can remain at far less than 5 meq of acid binding capacity. Also, in terms of product preparation, a retarded reaction and better compressibility into tablets is obtained. With the aid of this invention, an effervescent tablet can be prepared which for the first time contains an acid-sensitive drug, such as cisapride, or an H2 blocker such as cimetidine, and which has an acid-binding capacity of less than 5 meq for a tablet (or granular product) weight of only 1.6 to 2.3 g.

Further, in accordance with an especially advantageous embodiment of this invention, after the acid crystals have been covered with a coating of neutral substance, at least a portion of the carbonate and/or bicarbonate particles intended for a full dose can be applied to this coating, so that effervescent grains are formed from acid crystals on which a first coating of neutral substance has formed, and thereon a second coating of carbonate and/or bicarbonate, which has partially reacted with the acid in some cases.

The invention can be particularly expediently used for products or processes as described, for example, in EP-B1-76 340, US-A-4 867 942 and WO93/00886.

The application of the neutral substance, especially a sorbitol solution, for example, causes a depression of the melting point on the surface of the citric acid crystals. Thus, on the one hand, the adhesive force for the next coating containing alkali or alkaline earth carbonates and/or bicarbonates increases, and at the same time this signifies a slower and therefore more uniform reaction of the citric acid crystal surface and better passivation, so that the acid-sensitive drugs are less attacked by the effervescent grains. On the other hand, the melting point depression protracts the recrystallization time of the citric acid or of the citrates that have formed, which signifies better compressibility of the effervescent granules over a longer period of time.

The amount of neutral substance applied to the acid vehicle crystals depends on the amount of solvent with which the acid can be wetted, since a maximum of 50 - 70 % by weight can be dissolved in an aqueous solution. It is therefore preferably added in an amount of 0.05 bis 1.0, in particular 0.07 bis 0.8, % by weight, based on the acid. Additions of less than 0.07 have only a weak effect and those of less than 0.05 have no effect which is relevant according to the invention: the shelf-stability of acid-sensitive active substances is reduced. Additions of over 0.8 generally begin to have an interfering effect, and at above 1.0 the reactivity of citric acid and of the effervecent system is considerably slowed down.

However, this may be less troublesome in the case of granules since a longer dissolution time tends to be desirable there in order to allow the granules to sink on introduction into water and only thereafter to undergo a reaction for dissolution. Otherwise, however, the amounts of neutral substance which can be applied to, for example, citric acid are determined by the amount of solution with which the citric acid can be wetted, since the neutral substances are in fact applied in solution, and a 50 to max. 70% solution can be prepared. The citric acid crystals cannot be wetted with an infinitely large amount of water and hence solvent.

In certain circumstances, the neutral coating, especially if carbonate and/or bicarbonate particles are anchored on it, can also contain small amounts of a solid, edible organic acid, and in some cases an acid different from the one of which the vehicle crystals consist - as disclosed per se in another context - but here also in order to intensify the melting point depression and/or to control the effervescent reaction and rate of dissolution.

Each such effervescent grain is, taken by itself, actually a small effervescent "tablet", and effervesces by itself.

Therefore, if desired, a short dissolving time, small quantity and low acid-binding capacity can be achieved.

Experiments thus far towards achieving a fast-acting, small effervescent tablet by the use of monosodium citrate instead of citric acid have failed, because this greatly slows the effervescent reaction, since the monosodium citrate reacts more slowly with sodium bicarbonate, and such tablets usually have an acid consuming capacity exceeding 5 meq.

On the other hand, a very thin monosodium citrate coating in accordance with this invention, especially as a third or fourth layer, which can contain an additional neutral substance if desired, acts advantageously because 1 mol of monosodium citrate binds 1 mol of water of crystallization and thus contributes to the drying or to maintenance of dryness. Furthermore, in any case, uncovered citric acid surfaces can be covered again or more completely with bicarbonate.

Additionally, since many substances exhibit some form of taste sensation of which many are unpleasant, especially those exhibiting bitterness, it is desirable to keep the final effervescent solution, especially since it is in beverage form, within the pH range of 3.8 to 4.6. Experience has shown that within this range paricularly bitter substances can be more effectively masked.

While not obligatory, it is preferable to remove residual water from the reaction granules in the course of their preparation by a final treatment with alcohol. Alcohol may disrupt the bonding of water of crystallization, because during drying the residual moisture is removed along with the alcohol by evaporation. Small amounts of an antifoaming agent can also be added to the alcohol in order to accelerate the dissolution of the final tablet.

Many of the aforementioned drugs, especially cimetidine and cisapride, often cause frothing in an effervescent tablet. This is not due, however, to foaming such as that caused by tensides. That is to say, the active agents themselves, when stirred into water, do not foam. Instead, when the effervescent particles in the tablet dissolve, bubbles of carbon dioxide form.

These bubbles burst and leave the CO₂ on the surface. Now, if a less soluble or more hydrophobic substance is present, the undissolved particles envelop the CO₂ bubbles, and by forming such a film successfully prevent rapid bubble bursting, so that the bubbles with this film on the surface collect and thus a "foam" is formed. However, the "foam" already forming between the effervescent grains interferes with the continued reaction, and thus with the rapid dissolution of the tablet or granules. This circumstance is combatted according to the invention by the addition of very small amounts of at least one antifoaming agent with the result that any "foam" that forms as the effervescent reaction begins immediately collapses.

The antifoaming agent is preferably added in an amount of 0.005 to 0.5% by weight, based on the total amount including any fillers, flavors, etc., or 0.05 - 2.0% by weight, based on active substance. Additions of less than 0.005 have no effect relevant according to the invention; additions of more than 0.5 give rise to troublesome or unacceptable side effects.

In the case of active substances which are soluble, although not too freely soluble, as in the case of cimetidine, a percentage of simethicone of 0.1 - 0.3% by weight, based on active substance, is used, which is equivalent to the use of 0.016 - 0.028 percent (about 0.03%) based on the total tablet weight. The situation is somewhat different in the case of an insoluble hydrophobic active substance, such as cisapride (the monohydrate is used), where 1% of simethicone is used, based on the active substance, but an amount of 0.006% results when based on the tablet weight of 1.6 g. It is evident that the cisapride, as a slightly soluble, hydrophobic active substance, requires a larger amount of antifoaming agent for suppressing the foam, but the required fillers and the effervescent base result in a substantially smaller amount of simethicone being used per tablet, so that the ratios are inverted.

In the case of the soluble active substances, such as cimetidine and ranitidine, the simethicone is required in smaller amounts, in order to suppress the smaller tendency to foaming in the local reaction on dissolution of the effervescent tablet, whereas in the case of cisapride - as already mentioned - the tendency to foam is substantially greater and the principle is therefore also slightly different.

If larger amounts are used, film formation of simethicone occurs at the surface after dissolution of the effervescent tablet, by virtue of the fact that - especially in the case of insoluble active substances - particles of the active substance collect and remain hanging and thus result in unattractive dissolution behavior, this film then additionally having the tendency to form a ring on the glass wall.

In some cases, however, very small amounts of a tenside, for example, docusate sodium, are also added. Due to their wettable nature, such drug particles dissolve more quickly and no longer adhere to the foam bubbles. The proportion of such substances must be determined very precisely to achieve the desired dissolving characteristics.

Although in some cases the antifoaming agent can be applied to the effervescent system and/or to the drug, this is not preferred according to the invention. In the first case, it might cause undesirable slowing of the dissolution and reaction of the effervescent components unless very slight amounts of antifoaming agent sufficient for the achievement of the desired effect are used. In the second case, only those drugs are involved which, when the antifoaming agent is drawn onto them from a solution in a solvent (e.g., methyl ethyl ketone and acetone) at 40°C, do not lose any of their solubility or stability. Additionally, in the course of production with the use of finely powdered drugs the addition of antifoaming agents may lead to poor distribution because of drug particles attaching themselves to the antifoaming agent droplets.

It is therefore preferred, in accordance with this invention, that first the formation of a typical granular product from antifoaming agents and a neutral substance is undertaken, which product is thereafter mixed with the effervescent system and the drug, plus additional adjuvants if desired (e.g., perfumes, sweeteners and the like) and the mixture then compressed into tablet form.

The moisture released in the preparation of the effervescent system by the neutralization reaction, and not entirely removed by heating and/or vacuum treatment, as well as moisture picked up from the air during storage, can best be bound by the addition of a moisture-binding agent, especially anhydrous sodium carbonate (which can absorb 10 mols of water per mol) or sodium sulfate. The agent can be bound either by applying it to one or more of the coatings applied to the vehicle crystals, or by adding it to the total mixture. This improves shelf life because the reaction of the acid-sensitive active agent with the acid is further suppressed or completely prevented by the reduction of moisture. However, excessive amounts of such moisture-binding agent, for example sodium carbonate, are not desirable as it may retard the effervescent reaction.

Sodium carbonate as a drying agent, therefore, should not be used for completely covering the effervescent grains, since it is preferable to operate with only small quantities effective to merely dry the residual moisture, or to retard the reaction during manufacture, and to avoid undesirably lengthening the dissolving time of the tablet. Therefore, the final addition of sodium carbonate should not be used for complete coverage (or a tablet coating), due to both the quantity and the grain size (approx. 0.1 - 0.05 mm), and it is therefore not suitable for producing a continuous coating on the bicarbonate already present. However, it can be partially hooked onto the effervescent grains. It is also possible, however, not to add the sodium carbonate until after the drying operation.

In principle, the percentage amount of sodium carbonate per tablet depends on several factors, such as, for example, the amount of effervescent base used, the amount and type of the fillers used, the presence of other carbonates, such as, for example, calcium carbonate, etc.

The moisture-binding agent, in particular sodium carbonate, is preferably added in an amount of between 1 and 10, in particular 4 - 6, % by weight (based on the total amount, including any fillers, flavors, etc.). Additions of less than 4 have only a weak effect, and with those of less than 1, the drying effect and increase in stability is too small, they have no effect relevant according to the invention. Additions of over 6 generally begin to have a troublesome effect because sodium carbonate dissolves more slowly and reacts more poorly; above 10% the dissolution time is already significantly lengthened, since sodium carbonate first absorbs water (up to 10 molecules of water of crystallization) on dissolution of the effervescent tablet, i.e. is calcined and only then reacted with the citric acid.

Here it is to be emphasized that 1 mol of water of crystallization can be bound per mol by sodium citrate alone developing in or on the sorbitol layer, and in spite of any residual moisture present the sorbitol layer prevents or hinders any acid harm to the drug.

If all of the prescribed steps are followed in accordance with the invention, effervescent tablets can be produced, even with the difficult substances referred to, which at a tablet weight of, e.g., 1.6 g,will attain a dissolving time of less than 100 seconds. It is also to be noted that especially cimetidine, due to its hydrophobic character, further lengthens the dissolving time in comparison with other drugs, under otherwise equal conditions.

Granulation with sorbitol solution permits rapid dissolution without the incorporation of an extraneous acid that is otherwise necessary, for example, according to WO93/00886.

Furthermore, during the preparation of the effervescent systems of this invention, and in any case of the tablets themselves, the steps taken according to the invention will enable the control of reactions which take place at the surface of individual crystals or granules, which thus constitute a local mechanism, while also during dissolution the above-described desired advantages will be achieved throughout.

The system is also extraordinarily well suited for the processing of substances which are both acid-sensitive and sparingly soluble in water. Such substances, such as cisapride for example, exhibit very unpleasant behavior in suspension, since, as mentioned above, they tend to froth together with the effervescent system, adhere to a glass wall, form unpleasant rings and tend to agglomerate on the surface of the drink.

All the aforesaid problems can be effectively combatted by preparing seperate granules. For this purpose in yet another embodiment of this invention, there is provided a vehicle which can consist of an Aerosil and/or a neutral substance, on which the drug is applied preferably with the surface of its grains partially dissolved, and/or with binding agents and/or tensides if desired, and dried, or is bound to the vehicle surface by means of binders.

The amount of the suspended substance is limited to at most 8, preferably at most 4.5, % by weight (based on the total mixture), for example for cisapride, since larger amounts would result in increased sinking of the granule particles after dissolution of the tablet. On the other hand, the amount of binder used is likewise limited to 1% by weight, since it otherwise leads to undesirable agglomerated granules of active substance, suspended substance and binder, which dissolve only with difficulty and then sink to the bottom, i.e. prevent the desired suspension.

The invention will now be more fully described and understood with reference to the following examples of preferred embodiments.

Alternatively, the drug can also be dissolved in the methyl ethyl ketone or in acetone and coated onto mannitol, Aerosil^{(R)} and sodium bicarbonate.

### Example 1: Preparation of effervescent tablets containing 200 mg of cimetidine

### a) Preparation of the effervescent system

102 parts by weight of coarse citric acid and 25 parts by weight of finely powdered citric acid (the latter is preferable for improving build-up to effervescent grains on the vehicle crystal as the powder particles provide a rough surface on which up to about 30% of bicarbonate can be anchored) or tartaric acid are aspirated into a preheated vacuum tank and heated to approx. 60°C with stirring. Next, 0.85 parts by weight of a solution 1, which has been formed from 36 parts by weight each of water and sorbitol, 21 parts by weight of citric acid and 7 parts by weight of sodium bicarbonate, is aspirated and distributed on the citric acid by mixing. Thereafter, 52.5 parts by weight of sodium bicarbonate and 4.4 parts by weight of aspartame are added to this mixture, which is then stirred and dried by a vacuum of up to 200 mbar, after which 1.9 parts by weight of sodium carbonate are aspirated and distributed in the mixture by stirring, and the mixture is then dried by a vacuum of up to 15 mbar.

Next, a further 0.6 parts by weight of said solution are aspirated and distributed in the mixture by mixing. The resultant effervescent grains are dried in a vacuum of up to 20 mbar with stirring. If necessary, 0.25 parts by weight of 96% ethanol are also employed to dry the mixture, and aspirated. Then, again 9.3 parts by weight of sodium carbonate are bound onto the effervescent grain surface. After another final drying, the product is removed through a sieve.

### b) Preparation of the granulated antifoaming agent

In a vacuum mixing tank with a jacket temperature of 80°C, 7.7 parts by weight of sorbitol powder are added and heated to 50°C Then, 0.2 parts by weight of simethicone in a 30% butanone/acetone mixture (5:3) are aspirated in, stirred by vibrational mixing and dried under full vacuum down to 15 mbar at a temperature of at least 45°C.

### c) Preparation of the total mixture

In a mixer, 20 parts by weight of cimetidine, with 21.1 parts by weight of sorbitol powder if desired, are mixed for 10 minutes at 6 rpm with 178.4 parts by weight of the effervescent system prepared in a). Then 7 parts by weight of the antifoaming agent granules prepared in b) and screened through a 0.6 mm sieve, and 4.5 parts by weight of lemon flavoring, are added, mixed for another 5 minutes at 6 rpm. The final mixture is pressed into tablets which weigh 2.3 g, contain 200 mg of cimetidine, and have a hardness of 6-8 kp.

### Example 2: Preparation of effervescent tablets containing 200 mg of cimetidine, and citric and malic acid in the effervescent grains:

102 parts by weight of coarse citric acid, 25 parts by weight of powdered citric acid and 1.1 parts by weight of malic acid are heated to 60°C with stirring in a preheated vacuum tank. A solution consisting of 0.4 parts by weight of water, 0.22 parts by weight of sorbitol and 0.22 parts by weight of malic acid is then aspirated in and distributed onto the citric acid by mixing. 52.5 parts by weight of sodium bicarbonate and 4.4 parts by weight of aspartame are next added to the mixture and dried by stirring, in a vacuum of up to 200 mbar. Next, 1.9 parts by weight of sodium carbonate are aspirated in and distributed in the mixture by stirring, and then vacuum drying is performed down to 15 mbar. Finally, a final drying can be performed with ethanol, and then 9.3 parts by weight of sodium carbonate are added to the mixture. The rest of the procedure is similar to Example 1.

### Example 3: Effervescent tablets containing 400 mg of cimetidine, and mannitol as a neutral substance

49 parts by weight of citric acid are aspirated into a preheated vacuum tank and heated with stirring to 60°C. Then, 0.45 parts by weight of a solution 1, which has been prepared from 0.25 parts by weight of water and 0.20 parts by weight of mannitol, is aspirated in and distributed on the citric acid by mixing, whereupon 14.7 parts by weight of sodium bicarbonate and 3.2 parts by weight of aspartame are then added. Reaction is started with stirring and then drying is performed with a vacuum up to 200 mbar. 0.5 parts by weight of sodium carbonate are next aspirated and distributed in the mixture by stirring, and then drying is performed with a vacuum to 15 mbar. Then 0.5 parts by weight of a solution 2, which has been prepared from solution 1 by the addition of 0.16 parts by weight of monosodium citrate, is aspirated into the mixture and distributed by mixing. The effervescent grains obtained therefrom are then dried by vacuum and stirring to 20 mbar, and finally 2.8 parts by weight of sodium carbonate are added. To this mixture are then added 17.3 parts by weight of cimetidine, 4.3 parts by weight of mannitol, 8 parts by weight of sorbitol, 0.9 parts by weight of flavoring, and 4 parts by weight of antifoaming agent granules prepared according to Example 1 b), until distribution is uniform.

### Example 4: Effervescent tablets containing 300 mg of cimetidine, as well as maltodextrin as a neutral substance

Similarly to Example 3, for a 300 mg cimetidine effervescent tablet, a 50% solution of maltodextrin is selected, which is then used in the same amount as in the case of the 400 milligram form.

In all of the examples in which the tablets contain 100 to 400 mg of cimetidine, the tablet weight can be 2.3 g. The tablets have a dissolving time of preferably 60 to 150 seconds and a buffering capacity below 5 meq, measured according to USP XXII, by back-titration (with 0.5 N NaOH) of an effervescent tablet dissolved in 70 ml of water and with 30 ml of 1.0 N HCl added.

The figures given in the following table 1 are the percentages of individual ingredients in the particular total mixture of the illustrated preferred embodiments, which therefore are in the following preferred ranges:

**Table 1**

| Cimetidine | 2 - 30% | (corresponds to an effervescent tablet containing 50 to 600 mg of cimetidine) | |
|---|---|---|---|
| Citric acid | 30 - 60% | sorbitol | 5-20% |
| Sodium bicarbonate | 10 - 30% | mannitol | 2-10% |
| Sodium carbonate | 2 - 10% | simethicone | 0.005-0.5% |
| Aspartame | 1-4% | flavoring | 0.1-3% |

A preferred percentage composition of cimetidine effervescent tablets or bags of granules containing 100, 200, 300 and 400 mg of cimetidine, with a total weight of 2.31 grams, is summarized below in table 2:

**Table 2**

| | 100 mg | 200 mg | 300 mg | 400 mg |
|---|---|---|---|---|
| Cimetidine | 4.30 | 8.70 | 13 | 17.3 |
| Citric acid | 50 | 50 | 48.2 | 48.2 |
| Sodium citrate | 0.04 | 0.04 | 0.04 | 0.04 |
| Aspartame | 1.74 | 1.64 | 2.54 | 3.24 |
| Sorbitol | 12.5 | 12.5 | 12.8 | 8.00 |
| Sodium bicarbonate | 20.7 | 20.7 | 14.7 | 14.7 |
| Sodium carbonate | 4.4 | 4.4 | 3.5 | 3.3 |
| Manntiol | 4.3 | | 4.3 | 4.3 |
| HMA Lemon flavoring | 2.0 | 2.0 | 0.9 | 0.9 |
| Simethicone | 0.02 | 0.02 | 0.02 | 0.02 |

### Example 5: Cisapride effervescent tablets

### a) Preparation of the effervescent grains

655 parts by weight of crystalline citric acid, 70 parts by weight of citric acid powder and 8 parts by weight of sodium saccharin sodium are heated while mixing to 60°C. Then 2.8 parts by weight of a solution consisting of 0.6 parts by weight of sorbitol, 0.3 parts by weight of trisodium citrate, 0.5 parts by weight of citric acid and 1.6 parts by weight of water are aspirated into this mixture and distributed by mixing. Next, 340 parts by weight of sodium bicarbonate as well as 2 parts by weight of aspartame are added and reacted. Before drying, 77 parts by weight of sodium carbonate are added, whereupon the mixture is vacuum dried with slow stirring to 15 mbar.

### b) Preparation of the granulated drug

Insoluble and hydrophobic cisapride is attached to a suspending substance by means of a binder and a small amount of a tenside as follows: A solution of 10 parts by weight of cisapride, 2 parts by weight of polyvinylpyrrolidone and 0.8 part by weight of docusate sodium in 1 part by weight of ethanol and 40 parts by weight of acetone is applied to 10 parts by weight of Aerosil^{(R)}, uniformly distributed and then dried while stirring. The granules are sieved to 0.1 - 0.3 mm.

### c) Preparation of the end mixture

To 1152 parts by weight of effervescent grains are added 50 parts by weight of maltodextrin, 100 parts by weight of lactose, 184 parts by weight of mannitol, 40 parts by weight of flavoring, 50.2 parts by weight of anti-foaming granules (0.2 parts by weight of simethicone coated onto 50 parts by weight of mannitol), as well as the granulated drug prepared in b), mixing is carried out for 15 minutes for uniform distribution and the mixture is then pressed to form tablets of 1.6 g, which have an acid-binding capacity of only 2 meq. Cisapride effervescent tablets having such a low acid-binding capacity are unknown to date.

### Example 6: Beta-carotene effervescent tablets

With this extremely acid- and oxidation-sensitive aubstance, attention must be paid to an especially good covering of the acid. The surface and the contact zone on the beta-carotene must be kept alkaline. Therefore the effervescent grains are covered at least in part with calcium carbonate, thus insuring an alkaline surface. This, however, does result in a slightly longer dissolving time, which in this case is desirable, because the beta-carotene needs time to suspend while the tablet is dissolving. Large amounts of sorbitol, as in US-A-5 223 264 mentioned at the outset, are by no means suitable for a beta-carotene effervescent tablet which is intended to be dissolved or suspended in water.

### a) Preparation of the effervescent grains

1315 parts by weight of citric acid, 7 parts by weight of sodium saccharin and 45 parts by weight of sodium cyclamate are heated in a vacuum tank to 50°C. Then 16.8 parts by weight of a solution prepared from 3.6 parts by weight of calcium carbonate, 19 parts by weight of citric acid, 12 parts by weight of sorbitol, and 45 parts by weight of water are stirred in and distributed onto the citric acid by mixing. Next, 400 parts by weight of calcium carbonate and 190 parts by weight of citric acid are added and the mixture heated with stirring to 60°C. Then follows the second granulation with 44 parts by weight of the above-mentioned solution, and after distributing and mixing, 403 parts by weight of sodium bicarbonate are added, and also, before drying, 52 parts by weight of sodium carbonate. The mixture is then vacuum-dried to 15 mbar with slow mixing.

### b) Preparation of the end mixture

130 parts by weight of sorbitol and 540 parts by weight of mannitol and 50 parts by weight of flavoring, an encapsulated beta-carotene suspendable in water and corresponding to 2 to 15 parts by weight of 100% beta-carotene, plus, if desired, 50 to 250 parts by weight of vitamin C and/or a solid tocopheryl acetate suspendable in water (corresponding to 10 to 75 parts by weight of 100% tocopheryl acetate), plus still other vitamins if desired, are mixed with 2415 parts by weight of the effervescent grains prepared according to a). The product has a tablet weight of 3.3 g and its dissolving time is 60 to 90 seconds.

### Example 7: Ranitidine effervescent tablets

### a) Preparation of the effervescent grains

840 parts by weight of crystalline citric acid, 210 parts by weight of citric acid powder, 45 parts by weight of sodium cyclamate, and 4 parts by weight of sodium saccharin are heated in a vacuum mixing tank at 60°C. Then a solution consisting of 6 parts by weight of water, 1 part by weight of sodium citrate, and 3 parts by weight of sorbitol is aspirated in and distributed by stirring. 500 parts by weight of sodium bicarbonate are next added and allowed to react, and thereafter 370 parts by weight of monosodium citrate are added, which are also allowed to react. Lastly, 100 parts by weight of sodium carbonate are added and the granules are dried with slow stirring up to 15 mbar.

### b) Preparation of the end mixture

To the effervescent grains thus prepared, 167 parts by weight of ranitidine hydrochloride, 125 parts by weight of mannitol plus 100.4 parts by weight of a granulated antifoaming agent (consisting of 100 parts by weight of mannitol and 0.4 parts by weight of simethicone) and the flavoring agent are added. This mixture is mixed for 15 minutes for uniform distribution, and then pressed to tablets of 2.5 g. The tablets have a dissolving time of 60 to 80 seconds and an acid-binding capacity of about 2 meq and contain (in percent by weight) 6.8 ranitidine hydrochloride, 42.0 citric acid, 14.8 monosodium citrate, 20.0 sodium bicarbonate, 4.0 sodium carbonate, 2.0 sweeteners, 5.0 mannitol, 0.1 sorbitol, 4.0 granulated antifoaming agent (containing 0.016 diemthylpolysiloxane) and 1.2 flavoring.

### Example 8:

545 parts by weight of crystalline citric acid and 133 parts by weight of powdered citric or tartaric acid are mixed while heating to 60°C. Then, as the first coating, a solution which consists of 6 parts by weight of water and 4 parts by weight of sorbitol is distributed on the surface by stirring. Next, 222 parts by weight of sodium bicarbonate are made to react on the surface of the citric acid, and finally 80 parts by weight of sodium bicarbonate are added. The product is dried with slow stirring. The granules are screened to 1.5 mm, and then mixed for 10 minutes at 10 rpm with 167 parts by weight of ranitidine hydrochloride, 100 parts by weight of anti-foaming granules (containing 0.4 parts by weight of simethicone and 100 parts by weight of lactose), plus 54 parts by weight of sweetener and 40 parts by weight of flavoring, until uniform distribution is obtained. The mixture is then pressed to tablets weighing 1.43 g and having a dissolving time of 65-70 sec, a hardness of 8 kp, and an acid-binding capacity of about 1.5 meq. The product contains no monosodium citrate. Ranitidine effervescent tablets having such a low acid-binding capacity have not been disclosed to date.

### Example 9:

38.2% of citric acid is heated with 0.26% of sodium saccharin to 60°C, then two-thirds of a solution which consists of, with respect to the final mixture, 0.6% water, 0.18% sorbitol, and 0.12% sodium citrate are applied. The solution is distributed for 5 minutes by mixing at 10 rpm. Then 16.2% of sodium bicarbonate and 2.9% of aspartame are added and anchored on the surface of the citric acid by reaction on the neutral substance coating. Then follows a second wetting with the third one-third of the solution; then 12.9% monosodium citrate and, finally, 5.2% sodium carbonate are added. The effervescent grains are dried while mixing them slowly by applying a vacuum, at a temperature of at least 50°C, to 15 mbar. The basic effervescent granular product is screened to 1.5 mm and mixed with 11.0% of ranitidine hydrochloride, 6.5% of mannitol, 6.5% of anti-foaming granules plus 0.2% of flavoring, and pressed to tablets of 1.55 g, which have a dissolving time of 50 sec at a hardness of 7.3 kp and an acid-binding capacity of less than 2 meq.

### Example 10: Vehicle crystal grains coated only with a neutral substance

Since cisapride, for example, in comparison to ranitidine, is not as highly sensitive to acid, it is nevertheless also possible by the procedure to be described below to achieve protection against the acid, all the more so since the drug is embedded in granules.

### a) Preparation of the acid crystals coated with a neutral substance

593 parts by weight of crystalline citric acid plus 70 parts by weight of citric acid powder are heated to 60°C. Then a solution of 4 parts by weight of sorbitol in 4 parts by weight of water is applied and distributed onto the surface of the citric acid by mixing. Finally the citric acid thus coated is vacuum dried at 50 to 60°C.

In the case of both the form of effervescent product presented here and that of effervescent grains which contain a second alkali or alkali earth carbonate coating, it is possible to protect cisapride, for example, against attack by the citric acid in the drug granules by the addition of sodium bicarbonate.

### b) Preparation of the drug granules

160 parts by weight of mannitol, 10 parts by weight of cisapride, 5 parts by weight of aerosil and 10 parts by weight of sodium bicarbonate are heated with mixing to 60°C. Then half of a solution of 27 parts by weight of methyl ethyl ketone (or 45 parts by weight of acetone), 2 parts by weight of alcohol, 2 parts by weight of poly(vinyl pyrrolidone) K30, 1 part by weight of propylene glycol and 0.8 parts by weight of docusate sodium are added and distributed for 5 minutes for the purpose of uniform wetting. The mixture is dried to 0.8 bar, the second part of this solution is aspirated, and again distributed by stirring for 5-10 minutes, and finally vacuum dried.

The active agent granules are then screened to 0.3 mm and already have an enhanced protection against acid attack simply due to the sodium bicarbonate they contain. They can then be mixed with the acid crystals coated with neutral substance, the remaining carbonates and bicarbonates, as well as the other tablet ingredients, and pressed to give tablets.

### c) Preparation of the end mixture

The citric acid dried and coated according to a) is then mixed with the drug granules prepared according to b), 50 parts by weight of sweetener, 80 parts by weight of sodium carbonate, 430 parts by weight of sodium bicarbonate, and 50 parts by weight of maltodextrin, 100 parts by weight of lactose, 150 parts by weight of mannitol, 50 parts by weight of an antifoaming granulate, and 20 parts by weight of flavoring, and then pressed to tablets of about 1.6 g, which have a dissolving time of 60 to 70 seconds at a hardness of 7 kp.

### Example 11: Cisapride effervescent tablets

### a) Preparation of the effervescent granules:

Citric acid, consisting of an amount of 300 parts by weight of granules, 80 parts by weight of fine granules and 40 parts by weight of powder, together with 5 parts by weight of saccharin sodium, is uniformly wet at 60°C with 2.2 parts by weight of a solution which contains 0.4 part by weight of sorbitol, 0.15 part by weight of sodium bicarbonate, 0.45 part by weight of citric acid and 1.2 parts by weight of water. 12 parts by weight of malic acid are then aspirated in and uniformly anchored on the sorbitol layer formed on the citric acid crystals. Finally, 205 parts by weight of sodium bicarbonate and 1.2 parts by weight of aspartame are aspirated in and once again uniformly distributed. Finally, the material is covered with 46 parts by weight of sodium carbonate, vacuum-dried and discharged through a 1.2 mm sieve.

### b) Preparation of the active ingredient granules:

12 parts by weight of polyvinylpyrrolidone are dissolved in 12 parts by weight of ethanol; 6 parts by weight of propylene glycol and 6 parts by weight of docusate sodium are then added and the mixture is diluted with 165 parts by weight of ethyl methyl ketone. Half of this solution is distributed over a mixture of 960 parts by weight of mannitol, 30 parts by weight of Aerosil^{(R)}, 60 parts by weight of sodium bicarbonate and 61 parts by weight of cisapride, which is heated to 60°C. Partial drying is then carried out in vacuo, and further wetting is effected with the second half of the solution, followed by complete drying and dicharge through a 0.3 mm sieve.

The end mixture is prepared analogously to Example 5.

## Claims

1. A granular effervescent product suitable for preparing an aqueous solution or suspension of one or more pharmaceutically active substances for oral administration, being capable of being pressed into tablets, and/or said product in tablet form, comprising effervescent grains obtained from carrier crystals of at least one solid, edible organic acid which are substantially covered by at least one coating containing at least one water-soluble neutral substance, wherein said neutral substance is effective for depressing the melting point of the acid crystals on their surface, and at least one substance selected from the group consisting of alkali carbonate, alkali bicarbonate, alkaline earth carbonate, alkaline earth bicarbonate, alkali salt of at least one solid edible organic acid, alkaline earth salt of at least one solid edible organic acid is applied onto said coating.

2. The granular product or tablet according to claim 1, wherein the **neutral substance** is selected from the group consisting of a water-soluble polymer, a higher alcohol, a carbohydrate and a hydrocolloid, and which neutral substance is present in an amount of from about 0.05 to about 1.0 % by weight, preferably from about 0.07 to about 0.8 % by weight.

3. The granular product or tablet according to claim 1 or 2, wherein a **moisture-binding agent** is anchored on said effervescent grains, which moisture-binding agent preferably is selected from the group consisting of anhydrous sodium carbonate and sodium sulfate and preferably is applied in an amount of from about 4 to about 10 % by weight with respect to the total mixture.

4. The granular product or tablet according to any one of the preceding claims, wherein on the effervescent grains at least one **additional coating** is applied, comprising a substance selected from the group consisting of alkali salts and/or alkaline earth salts of at least one solid, edible, organic acid as buffer and, optionally, comprising an additional neutral substance, and wherein preferably at least one of the coatings contains an antifoaming agent.

5. The granular effervescent product or tablet according to any one of the preceding claims, wherein the granular product, or said granular product compressed in tablet form, further comprises at least one **antifoaming agent** present in a granular product of its own.

6. The granular product or tablet according to claim 4 or 5, wherein the antifoaming agent is selected from the group consisting of dimethicone and simethicone and is applied in an amount of from about 0.005 to about 0.5 % by weight with respect to the total mixture or from about 0.05 to about 2.0 % by weight with respect to the pharmaceutically active substance.

7. The granular product or tablet according to any one of the preceding claims, wherein it has an **acid-binding capacity** of less than 5, preferably less than 3 meq, measured according to USP XXII.

8. The granular product or tablet according to any one of the preceding claims, wherein, at a **total weight** of no more than 2.5, preferably no more than 2.0 grams, in water at room temperature, it has a **dissolving time** of less than 180, preferably less than 120 seconds.

9. The granular product or tablet according to any one of the preceding claims, comprising a pharmaceutically active substance which is hydrophobic and wherein the **hydrophobic substance** is present in **granules separate** from the effervescent components, in which granules the hydrophobic substance is coated or anchored onto at least one substance selected from the group consisting of suspending agents - preferably selected from the group consisting of Aerosil^{(R)} and Avicel^{(R)} - and neutral substances - preferably selected from the group consisting of mannitol and sorbitol.

10. The granular product or tablet according to claim 9, wherein the granules also contain at least one component selected from the group consisting of **binders** - preferably polyvinylpyrrolidone (PVP) -, small amounts of a **tenside** -preferably selected from the group consisting of dioctyl sodium sulfosuccinate and sodium lauryl sulfate -, alkali and/or alkaline earth carbonate and/or **bicarbonate**.

11. The granular product or tablet according to any one of the preceding claims, wherein it contains, with respect to the total mixture, about 2 to about 30 % by weight of **cimetidine**; about 30 to about 60 % by weight of a solid, edible organic acid; about 12 to about 40 % by weight of at least one alkali or alkaline earth carbonate or bicarbonate (of which about 2 to about 10 % by weight is sodium carbonate as moisture-binding agent); about 1 to about 4 % by weight of a sweetener; about 0.01 to about 30 % by weight of a neutral substance (of which about 0.01 to about 0.05 % by weight is for the neutral substance coating), preferably about 3 to about 20 % by weight of sorbitol and about 2 to about 10 % by weight of mannitol; about 0.005 to about 0.5 % by weight of an antifoaming agent, and about 0.1 to about 3 % by weight of flavoring agent.

12. The granular product or tablet according to any one of claims 1 to 10, wherein it contains, with respect to the total mixture, the following components: about 0.4 to about 4.5 % by weight of **cisapride**; about 0.4 to about 4.5 % by weight of suspending agent; about 0.1 to about 1 % by weight of binder, preferably polyvinylpyrrolidone (PVP); about 0.03 to about 0.35 % by weight of tenside, preferably dioctyl sodium sulfosuccinate; about 30 to about 55 % by weight of a solid, edible organic acid, preferably citric acid; about 12 to about 40 % by weight of at least one alkali or alkaline earth carbonate or bicarbonate (of which about 2 to about 10 % by weight is sodium carbonate as moisture-binding agent); about 0.3 to about 2.5 % by weight of sweetener; about 0.02 to about 55 % by weight of neutral substance (of which about 0.02 to about 0.1 % by weight is for the neutral substance coating), preferably selected from the group consisting of maltodextrin, lactose and mannitol; about 0.005 to about 0.05 % by weight of antifoaming agent, preferably selected from the group consisting of dimethicone and simethicone; and about 0.2 to about 5 % by weight of flavouring.

13. The granular product or tablet according to any one of claims 1 to 10, wherein it contains, with respect to the total mixture, the following components:
- about 0.1 to about 0.5 % by weight of **beta-carotene** (100%);
- about 0 to about 2 % by weight of tocopheryl acetate (100%);
- about 35 to about 70 % by weight of solid, edible organic acid, preferably about 0 to about 10 % by weight of ascorbic acid, about 35 to about 55 % by weight of citric acid, and about 0 to about 5 % by weight of malic acid;
- about 11 to about 38 % by weight of at least one alkali or alkaline earth carbonate or bicarbonate, preferably about 5 to about 15 % by weight of calcium carbonate and about 5 to about 20 % by weight of sodium bicarbonate;
- about 1 to about 4 % by weight of sweetener;
- about 0.1 to about 35.0 % by weight of neutral substance (of which about 0.1 to about 0.5 % by weight is for the neutral substance coating), preferably about 1 to about 10 % by weight of sorbitol and about 5 to about 25 % by weight of mannitol; and
- about 0.3 to about 3 % by weight of flavouring.

14. The granular product or tablet according to any one of claims 1 to 10, wherein it contains, with respect to the total mixture, the following components : about 3 to about 14 % by weight of **ranitidine** hydrochloride (75 - 300 mg per dose); about 30 to about 50 % by weight of citric acid; about 0 to about 20 % by weight of monosodium citrate; about 10 to about 30 % by weight of sodium bicarbonate; about 2 to about 10 % by weight of sodium carbonate; about 1 to about 3 % by weight of sweetener; about 0.05 to about 0.2 % by weight of neutral substance for the first coating as well as about 0 to about 15 % by weight of additional neutral substances; about 0 to about 8 % by weight of antifoaming granules, and about 0.1 to about 4 % by weight of flavoring.

15. An effervescent tablet containing at least one pharmaceutically active substance and an effervescent system comprising at least one solid, edible, organic acid, at least one alkali metal carbonate or bicarbonate as a gas-forming component and at least one alkali metal salt of the acid, **at least two layers** being applied to carrier crystals consisting of the at least one acid, wherein the first layer contains at least one other, solid, edible, organic acid or the alkali metal salt of this other acid, or both, whereas the second layer contains at least one alkali metal salt of said at least one acid, and wherein the first layer additionally contains a neutral substance selected from the group consisting of a water-soluble polymer, a higher alcohol, a carbohydrate and a hydrocolloid.

16. A granular product or tablet with an effervescent system according to any one of claims 1 - 15 and **cisapride** as the pharmaceutically active substance, wherein, at a total weight of less than 2 grams, preferably less than about 1.6 grams, it has an acid-binding capacity of less than 5 meq, preferably less than 3 meq.

17. A granular product or tablet with an effervescent system according to any one of claims 1 - 15 and **cimetidine** as the pharmaceutically active substance, wherein, at a total weight of less than 2.5 grams, preferably less than about 2.0 grams, it has an acid-binding capacity of less than 5 meq, preferably less than 3 meq.

18. A granular product or tablet with an effervescent system according to any one of claims 1 - 15 and **ranitidine** as the pharmaceutically active substance, wherein, at a total weight of less than 2.6 grams, preferably less than 2.0 g, it has an acid-binding capacity of less than 3 meq, preferably less than 2 meq.

19. A method for the preparation of a granular product or of a tablet according to any one of the preceding claims, wherein crystals of at least one solid, edible organic acid are wetted with an aqueous solution of a neutral substance, and then, before complete drying, an alkali and/or alkaline earth carbonate and/or bicarbonate in powder form is uniformly distributed and anchored on the moist surface layer by mixing, whereupon the effervescent grains thus prepared are dried and mixed with a pharmaceutically active substance - preferably with an acid-sensitive one, especially one that is selected from the group consisting of H2-blockers, **cimetidine**, **ranitidine**, **cisapride and beta-carotene** - and pharmaceutically acceptable adjuvants, and optionally compressed into tablets.

20. The method according to claim 19, wherein, on the effervescent grains, at least one **additional coating** is applied by wetting the grains with the solution of a buffer substance, preferably one that is selected from the group consisting of alkali carbonate, alkali bicarbonate, alkaline earth carbonate, alkaline earth bicarbonate, alkali salt of at least one solid edible organic acid and alkaline earth salt of at least one solid edible organic acid.

21. The method according to claim 19 or 20, wherein the solution further comprises a **neutral substance** selected from the group consisting of a water-soluble polymer, a higher alcohol, a carbohydrate and a hydrocolloid.

22. The method according to any one of claims 19 to 21, wherein, in addition to the drug, the effervescent grains are also mixed with a **granular product** which has been made by applying an antifoaming agent in an appropriate solvent to the surface of neutral substance particles, and drying the solvent.

23. The method according to any one of claims 19 to 22, wherein the dried effervescent **grains are wetted** with ethanol, which preferably contains an **antifoaming agent** dissolved, and are dried again, by evaporating the ethanol, to remove residual moisture.

24. The method according to any one of claims 19 to 23, wherein the pharmaceutically active substance, before admixing it to the effervescent system, is - together with a **binding agen**t and/or a **tenside** - applied in solution to and uniformly distributed on the grains of a **suspension agent** and dried.

25. The method according to any one of claims 19 to 24, wherein the pharmaceutically active substance, before admixing it to the effervescent system, is mixed with at least one neutral substance, at least one suspension agent and at least one substance selected from the group of alkali carbonate, alkali bicarbonate, alkaline earth carbonate, alkaline earth bicarbonate, alkali salt of at least one solid edible organic acid, alkaline earth salt of at least one solid edible organic acid, whereafter a solution of at least one binding agent and/or a tenside is at least once applied to, distributed on the grains of the mixture and dried.

26. A process for the manufacture of effervescent granules from a powdered or granular mixture of a solid, edible, organic acid and the carbonate and/or bicarbonate of an alkali and/or alkaline earth metal under vacuum, wherein, for the passivation of the surface of at least one of the components to a state of strong inertia to the reaction, there is added to the heated mixture during the treatment under vacuum a metered quantity of a polar solvent, the difference in pressure caused by development of carbon dioxide through the addition of solvent during the reaction being determined up to a maximum of 1000 mbar, the volume and mass of the carbon dioxide liberated being ascertained from this difference in pressure, and the heat treatment being repeated, after rapid drying of the mixture, as many times as necessary to obtain passivation of the surface as indicated by an evident slowing down of the reaction and by a reduced development of gas, and wherein in said polar solvent there is dissolved a neutral substance selected from the group consisting of a water-soluble polymer, a higher alcohol, a carbohydrate and a hydrocolloid.

27. A process for the preparation of an effervescent granular material containing at least one solid, crystalline edible organic acid and at least one carbonate of an alkali metal or an alkaline earth metal which splits off CO₂ upon reaction with said organic acid in aqueous solution, which comprises:
- pre-reacting a portion of said organic acid and said carbonate in solution in water and/or alcohol to form a prereaction product,
- adding said pre-reaction product to an additional portion of said organic acid in crystalline form with thorough mixing to form a first coating by reaction with said organic acid crystals and liberation of the resulting water of crystallization,
- applying at least one additional coating including said carbonate onto the organic acid crystals with said first coating adhering thereto, and
- terminating the reaction after the last coating has been applied by drying, wherein to said pre-reaction product there is added a neutral substance selected from the group consisting of a water-soluble polymer, a higher alcohol, a carbohydrate and a hydrocolloid.

## Patentansprüche

1. Ein granuläres Brauseprodukt, welches zum Herstellen einer wässrigen Lösung oder Suspension einer oder mehrerer pharmazeutisch aktiver Substanzen zur oralen Verabreichung geeignet ist, und welches in Tabletten pressbar ist, und/oder dieses Produkt in Tablettenform, mit Brausekörnern, die von Trägerkristallen wenigstens einer festen, geniessbaren organischen Säure erhalten worden sind, welche im wesentlichen mit wenigstens einer Beschichtung bedeckt sind, die mindestens eine wasserlösliche, neutrale Substanz enthält, wobei die neutrale Substanz zum Absenken des Schmelzpunktes der Säurekristalle an ihrer Oberfläche wirksam ist, und wenigstens eine aus der aus Alkalicarbonat, Alkalibicarbonat, Erdalkalicarbonat, Erdalkalibicarbonat, einem Alkalisalz und einem Erdalkalisalz zumindest einer festen, geniessbaren organischen Säure bestehenden Gruppe wenigstens einer festen, geniessbaren organischen Säure ausgewählte Substanz auf der Beschichtung angebracht ist.

2. Granuläres Produkt oder Tablette nach Anspruch 1, wobei die **neutrale Substanz** aus der aus einem wasserlöslichen Polymer, einem höheren Alkohol, einem Kohlehydrat und einem Hydrokolloid bestehenden Gruppe ausgewählt ist, welche neutrale Substanz in einer Menge von etwa 0,05 bis annähernd 1,0 Gewichts-%, vorzugsweise von etwa 0,07 bis ungefähr 0,8 Gewichts-%, vorhanden ist.

3. Granuläres Produkt oder Tablette nach Anspruch 1 oder 2, wobei ein **Feuchtigkeitsbindemittel** an den Brausekörnern verankert ist, welches Feuchtigkeitsbindemittel vorzugsweise aus der aus kalziniertem Soda und Natriumsulfat bestehenden Gruppe ausgewählt ist und vorzugsweise in einer Menge von etwa 4 bis ungefähr 10 Gewichts-%, bezogen auf die gesamte Mischung, eingesetzt ist.

4. Granuläres Produkt oder Tablette nach einem der vorhergehenden Ansprüche, wobei wenigstens eine **zusätzliche Beschichtung** an den Brausekörnern angebracht ist, welche eine aus der aus Alkalisalzen und/oder Erdalkalisalzen wenigstens einer festen, geniessbaren organischen Säure bestehenden Gruppe ausgewählte Substanz als Puffer und gegebenenfalls eine zusätzliche neutrale Substanz aufweist, und wobei vorzugsweise wenigstens eine der Beschichtungen ein Antischaummittel enthält.

5. Granuläres Produkt oder Tablette nach einem der vorhergehenden Ansprüche, wobei das granuläre Produkt oder das in Tablettenform gepresste granuläre Produkt ferner mindestens ein in einem eigenen granulären Produkt vorhandenes **Antischaummittel** aufweist.

6. Granuläres Produkt oder Tablette nach Anspruch 4 oder 5, wobei das Antischaummittel aus der aus Dimethicon und Simethicon bestehenden Gruppe ausgewählt ist und in einer Menge von etwa 0,005 bis ungefähr 0,5 Gewichts-%, bezogen auf die gesamte Mischung, oder von etwa 0,05 bis ungefähr 2,0 Gewichts-%, bezogen auf die pharmazeutisch aktive Substanz, eingesetzt ist.

7. Granuläres Produkt oder Tablette nach einem der vorhergehenden Ansprüche, wobei es bzw. sie eine **Säurebindungsfähigkeit** von weniger als 5, vorzugsweise weniger als 3 meq, gemessen nach USP XXII, aufweist.

8. Granuläres Produkt oder Tablette nach einem der vorhergehenden Ansprüche, wobei es bzw. sie bei einem **Gesamtgewicht** von nicht mehr als 2,5, vorzugsweise nicht mehr als 2,0 Gramm in Wasser bei Raumtemperatur eine **Auflösungszeit** von weniger als 180, vorzugsweise weniger als 120, Sekunden aufweist.

9. Granuläres Produkt oder Tablette nach einem der vorhergehenden Ansprüche, mit einer hydrophoben pharmazeutisch aktiven Substanz, wobei die **hydrophobe Substanz** in von den Brausekomponenten gesonderten Granula vorliegt, in welchen Granula die hydrophobe Substanz auf wenigstens einer aus der aus Suspendiermitteln - welche vorzugsweise aus der aus Aerosil® und Avicel® bestehenden Gruppe gewählt sind - und neutralen Substanzen - welche vorzugsweise aus der aus Mannitol und Sorbitol bestehenden Gruppe gewählt sind - bestehenden Gruppe ausgewählten Substanz geschichtet bzw. an ihnen verankert ist.

10. Granuläres Produkt oder Tablette nach Anspruch 9, wobei die Granula auch wenigstens eine aus der aus **Bindern** - vorzugsweise Polyvinylpyrrolidon (PVP) -, geringen Mengen eines **Tensids** - welches vorzugsweise aus der aus Dioctyl-Natriumsulfosuccinat und Natriumlaurylsulfat bestehenden Gruppe gewählt ist - , Alkali- und/oder Erdalkalicarbonat und/oder -**bicarbonat** bestehenden Gruppe ausgewählte Komponente enthalten.

11. Granuläres Produkt oder Tablette nach einem der vorhergehenden Ansprüche, wobei es bzw. sie, bezogen auf die gesamte Mischung, etwa 2 bis ungefähr 30 Gewichts-% **Cimetidin**; etwa 30 bis ungefähr 60 Gewichts-% einer festen, geniessbaren organischen Säure, etwa 12 bis ungefähr 40 Gewichts-% wenigstens eines Alkali- oder Erdalkalicarbonats oder -bicarbonats (wovon etwa 2 bis ungefähr 10 Gewichts-% Natriumcarbonat als Feuchtigkeitsbindemittel ist); etwa 1 bis ungefähr 4 Gewichts-% eines Süssstoffes; etwa 0,01 bis ungefähr 30 Gewichts-% einer neutralen Substanz (wovon etwa 0,01 bis ungefähr 0,05 Gewichts-% für die Beschichtung mit neutraler Substanz ist), vorzugsweise etwa 3 bis ungefähr 20 Gewichts-% Sorbitol und etwa 2 bis ungefähr 10 Gewichts-% Mannitol; etwa 0,005 bis ungefähr 0,5 Gewichts-% eines Antischaummittels und etwa 0,1 bis ungefähr 3 Gewichts-% eines Geschmacksmittels.

12. Granuläres Produkt oder Tablette nach einem der Ansprüche 1 bis 10, wobei es bzw. sie, bezogen auf die gesamte Mischung, die folgenden Komponenten enthält: etwa 0,4 bis ungefähr 4,5 Gewichts-% **Cisaprid**; etwa 0,4 bis ungefähr 4,5 Gewichts-% eines Suspendiermittels; etwa 0,1 bis ungefähr 1 Gewichts-% Binder, vorzugsweise Polyvinylpyrrolidon (PVP); etwa 0,03 bis ungefähr 0,35 Gewichts-% Tensid, vorzugsweise Dioctyl-Natriumsulfosuccinat; etwa 30 bis ungefähr 55 Gewichts-% einer festen, geniessbaren organischen Säure, vorzugsweise Zitronensäure; etwa 12 bis ungefähr 40 Gewichts-% wenigstens eines Alkali- und/oder Erdalkalicarbonats oder - bicarbonats (wovon etwa 2 bis ungefähr 10 Gewichts-% Natriumcarbonat als Feuchtigkeitsbindemittel sind); etwa 0,3 bis ungefähr 2,5 Gewichts-% eines Süssstoffes; etwa 0,02 bis ungefähr 55 Gewichts-% einer neutralen Substanz (wovon etwa 0,02 bis ungefähr 0,1 Gewichts-% für die Beschichtung mit neutraler Substanz ist), die vorzugsweise aus der aus Maltodextrin, Laktose und Mannitol bestehenden Gruppe ausgewählt ist; etwa 0,005 bis ungefähr 0,05 Gewichts-% eines Antischaummittels, welches vorzugsweise aus der aus Dimethicon und Simethicon bestehenden Gruppe ausgewählt ist; und etwa 0,2 bis ungefähr 5 Gewichts-% eines Geschmacksmittels.

13. Granuläres Produkt oder Tablette nach einem der Ansprüche 1 bis 10, wobei es bzw. sie, bezogen auf die gesamte Mischung, die folgenden Komponenten enthält:
- etwa 0,1 bis ungefähr 0,5 Gewichts-% **beta-Carotin** (100%);
- etwa 0 bis ungefähr 2 Gewichts-% Tocopherylazetat (100%);
- etwa 35 bis ungefähr 70 Gewichts-% einer festen, geniessbaren organischen Säure, vorzugsweise etwa 0 bis ungefähr 10 Gewichts-% Ascorbinsäure, etwa 35 bis ungefähr 55 Gewichts-% Zitronensäure und etwa 0 bis ungefähr 5 Gewichts-% Maleinsäure;
- etwa 11 bis ungefähr 38 Gewichts-% wenigstens eines Alkali- oder Erdalkalicarbonats oder -bicarbonats, vorzugsweise etwa 5 bis ungefähr 15 Gewichts-% Calciumcarbonat und etwa 5 bis ungefähr 20 Gewichts-% Natriumbicarbonat;
- etwa 1 bis ungefähr 4 Gewichts-% eines Süssstoffes;
- etwa 0,1 bis ungefähr 35,0 Gewichts-% einer neutralen Substanz (wovon etwa 0,1 bis ungefähr 0,5 Gewichts-% für die Beschichtung mit neutraler Substanz ist), die vorzugsweise etwa 1 bis ungefähr 10 Gewichts-% Sorbitol und etwa 5 bis ungefähr 25 Gewichts-% Mannitol sind; und
- etwa 0,3 bis ungefähr 3 Gewichts-% eines Geschmacksmittels.

14. Granuläres Produkt oder Tablette nach einem der Ansprüche 1 bis 10, wobei es bzw. sie, bezogen auf die gesamte Mischung, die folgenden Komponenten enthält: etwa 3 bis ungefähr 14 Gewichts-% **Ranitidin**-Hydrochlorid (75 - 300 mg pro Dosis); etwa 30 bis ungefähr 50 Gewichts-% Zitronensäure; etwa 0 bis ungefähr 20 Gewichts-% Natriummonozitrat; etwa 10 bis ungefähr 30 Gewichts-% Natriumbicarbonat; etwa 2 bis ungefähr 10 Gewichts-% Natriumcarbonat; etwa 1 bis ungefähr 3 Gewichts-% eines Süssstoffes; etwa 0,05 bis ungefähr 0,2 Gewichts-% einer neutralen Substanz für die erste Beschichtung sowie etwa 0 bis ungefähr 15 Gewichts-% zusätzlicher neutraler Substanzen; etwa 0 bis ungefähr 8 Gewichts-% Antischaumgranula, und etwa 0,1 bis ungefähr 4 Gewichts-% eines Geschmacksmittels.

15. Eine Brausetablette, welche wenigstens eine pharmazeutisch aktive Substanz und ein Brausesystem mit wenigstens einer festen, geniessbaren organischen Säure, wenigstens einem Alkalimetallcarbonat oder -bicarbonat als gasbildende Komponente und mindestens einem Alkalimetallsalz der Säure, wobei **zumindest zwei Schichten** auf Trägerkristalle aufgetragen sind, welche aus der wenigstens einen Säure bestehen, wobei die erste Schicht zumindest eine weitere feste, geniessbare organische Säure oder das Alkalimetallsalz dieser weiteren Säure oder beide enthält, wogegen die zweite Schicht mindestens ein Alkalimetallsalz der wenigstens einen Säure enthält, und wobei die erste Schicht zusätzlich eine aus der aus einem wasserlöslichen Polymer, einem höheren Alkohol, einem Kohlehydrat und einem Hydrocolloid bestehenden Gruppe ausgewählte neutrale Substanz enthält.

16. Granuläres Produkt oder Tablette mit einem Brausesystem nach einem der Ansprüche 1 bis 15 und **Cisaprid** als pharmazeutisch aktive Substanz, wobei, bei einem Gesamtgewicht von weniger als 2 Gramm, vorzugsweise weniger als etwa 1,6 Gramm, es bzw. sie eine Säurebindungsfähigkeit von weniger als 5 meq, vorzugsweise weniger als 3 meq, besitzt.

17. Granuläres Produkt oder Tablette mit einem Brausesystem nach einem der Ansprüche 1 bis 15 und **Cimetidin** als pharmazeutisch aktive Substanz, wobei, bei einem Gesamtgewicht von weniger als 2,5 Gramm, vorzugsweise weniger als etwa 2,0 Gramm, es bzw. sie eine Säurebindungsfähigkeit von weniger als 5 meq, vorzugsweise weniger als 3 meq, besitzt.

18. Granuläres Produkt oder Tablette mit einem Brausesystem nach einem der Ansprüche 1 bis 15 und **Ranitidin** als pharmazeutisch aktive Substanz, wobei, bei einem Gesamtgewicht von weniger als 2,6 Gramm, vorzugsweise weniger als 2,0 Gramm, es bzw. sie eine Säurebindungsfähigkeit von weniger als 3 meq, vorzugsweise weniger als 2 meq, besitzt.

19. Verfahren zur Herstellung eines granulären Produktes oder einer Tablette nach einem der vorhergehenden Ansprüche, bei dem Kristalle wenigstens einer festen, geniessbaren organischen Säure mit einer wässrigen Lösung einer neutralen Substanz angefeuchtet wird und dann vor dem vollständigen Trocknen ein Alkali- und/oder Erdalkalicarbonat und/oder -bicarbonat in Pulverform gleichmässig verteilt und an der feuchten Oberflächenschicht durch Mischen verankert wird, worauf die so hergestellten Brausekörner getrocknet und mit einer pharmazeutisch aktiven Substanz - vorzugsweise mit einer säureempfindlichen, insbesondere einer aus der aus H2-Blockern, **Cimetidin, Ranitidin, Cisaprid und beta-Carotin** bestehenden Gruppe ausgewählten - und pharmazeutisch akzeptablen Hilfsmitteln gemischt, und gegebenenfalls zu Tabletten gepresst, werden.

20. Verfahren nach Anspruch 19, bei dem auf den Brausekörnern mindestens eine **zusätzliche Beschichtung** durch Befeuchten der Körner mit der Lösung einer Puffersubstanz aufgebracht wird, vorzugsweise einer solchen, welche aus der aus Alkalicarbonat, Alkalibicarbonat, Erdalkalicarbonat, Erdalkalibicarbonat, einem Alkalisalz zumindest einer festen, geniessbaren organischen Säure und einem Erdalkalisalz zumindest einer festen, geniessbaren organischen Säure bestehenden Gruppe ausgewählt ist.

21. Verfahren nach Anspruch 19 oder 20, bei dem die Lösung ferner eine aus der aus einem wasserlöslichen Polymer, einem höheren Alkohol, einem Kohlehydrat und einem Hydrocolloid bestehenden Gruppe ausgewählt ist.

22. Verfahren nach einem der Ansprüche 19 bis 21, bei dem, zusätzlich zum Arzneimittel, die Brausekörner auch mit einem **granulären Produkt** gemischt werden, das durch Auftragen eines Antischaummittels in einer geeigneten Lösung auf die Oberfläche von Partikeln einer neutralen Substanz hergestellt worden ist, und das Lösungsmittel getrocknet wird.

23. Verfahren nach einem der Ansprüche 19 bis 22, bei dem die getrockneten **Brausekörner** mit Äthanol **befeuchtet**, das vorzugsweise ein **Antischaummittel** gelöst enthält, und durch Verdampfen des Äthanols wieder getrocknet werden, um die Restfeuchtigkeit zu beseitigen.

24. Verfahren nach einem der Ansprüche 19 bis 23, bei dem die pharmazeutisch aktive Substanz, vor ihrer Zumischung zum Brausesystem, in Lösung - zusammen mit einem **Bindemittel** und/oder einem **Tensid** - auf die Körner eines **Suspendiermittels** aufgetragen und gleichmässig verteilt und getrocknet wird.

25. Verfahren nach einem der Ansprüche 19 bis 24, bei dem die pharmazeutisch aktive Substanz, vor ihrer Zumischung zum Brausesystem, mit wenigstens einer neutralen Substanz, mindestens einem Suspendiermittel und zumindest einer aus der aus Alkalicarbonat, Alkalibicarbonat, Erdalkalicarbonat, Erdalkalibicarbonat, einem Alkalisalz zumindest einer festen, geniessbaren organischen Säure und einem Erdalkalisalz zumindest einer festen, geniessbaren organischen Säure bestehenden Gruppe ausgewählten Substanz gemischt wird, worauf die Lösung wenigstens eines Bindemittels und/oder eines Tensids zumindest einmal auf die Körner der Mischung aufgetragen, verteilt und getrocknet wird.

26. Verfahren zur Herstellung von Brausegranula aus einer pulverförmigen oder granulären Mischung einer festen, geniessbaren organischen Säure und dem Carbonat und/oder Bicarbonat eines Alkali- und/oder Erdalkalimetalls unter Vakuum, bei dem zur Passivierung der Oberfläche wenigstens einer der Komponenten zu einem Zustand starker Trägheit gegenüber der Reaktion der erhitzten Mischung während der Behandlung unter Vakuum eine dosierte Menge eines polaren Lösungsmittels zugefügt wird, die durch die Entwicklung von Kohlendioxyd durch die Zugabe des Lösungsmittels während der Reaktion verursachte Druckdifferenz bis auf ein Maximum von 1000 bar bestimmt wird, wobei das Volumen und die Masse des freigesetzten Kohlendioxyds aus dieser Druckdifferenz ermittelt wird, und die Wärmebehandlung nach raschem Trocknen der Mischung so oft wiederholt wird, als notwendig ist, um die Passivierung der Oberfläche zu erhalten, wie durch eine deutliche Verlangsamung der Reaktion und eine verringerte Gasentwicklung angezeigt wird, und wobei in der polaren Lösung eine aus der aus einem wasserlöslichen Polymer, einem höheren Alkohol, einem Kohlehydrat und einem Hydrocolloid bestehenden Gruppe ausgewählte neutrale Substanz gelöst wird.

27. Verfahren zur Herstellung von granulärem Brausematerial, welches mindestens eine feste, geniessbare organische Säure und zumindest ein Carbonat eines Alkali- oder Erdalkalimetalls enthält, das bei Reaktion mit der organischen Säure in einer wässrigen Lösung CO₂ abgibt, welches folgendes aufweist:
- vorab Umsetzen eines Teiles der organischen Säure und des Carbonats in einer Lösung in Wasser und/oder Alkohol, um ein Vorreaktionsprodukt zu schaffen,
- Zugeben des Vorreaktionsproduktes zu einem weiteren Teil der organischen Säure in kristalliner Form unter sorgfältigem Mischen, um durch Reaktion mit den Kristallen der organischen Säure und der sich daraus ergebenden Freisetzung von Kristallisationswasser eine erste Beschichtung zu bilden,
- Aufbringen wenigstens einer weiteren, das Carbonat aufweisenden Beschichtung auf den Kristallen der organischen Säure, an denen die erste Beschichtung anhaftet, und
- Abschliessen der Reaktion, nachdem die letzte Beschichtung aufgetragen worden ist, durch Trocknen, wobei eine aus der aus einem wasserlöslichen Polymer, einem höheren Alkohol, einem Kohlehydrat und einem Hydrocolloid bestehenden Gruppe ausgewählte neutrale Substanz dem Vorreaktionsprodukt hinzugefügt wird.

## Revendications

1. Produit effervescent granulé, convenant pour la préparation d'une suspension ou d'une solution aqueuse d'une substance active du point de vue pharmaceutique ou davantage, destiné à une administration orale, et susceptible d'être pressé en comprimés et/ou ledit produit sous forme de comprimés, comprenant des grains effervescents obtenus à partir de cristaux porteurs d'au moins un acide organique alimentaire et solide, qui sont sensiblement recouverts par au moins un revêtement contenant au moins une substance neutre hydrosoluble, dans lequel ladite substance neutre est capable d'abaisser le point de fusion des cristaux d'acide à leur surface, et au moins une substance - choisie dans le groupe constitué par les carbonates alcalins, les bicarbonates alcalins, les carbonates alcalino-terreux, les bicarbonates alcalino-terreux et les sels alcalins d'au moins un acide organique alimentaire et solide et les sels alcalino-terreux d'au moins un acide organique alimentaire et solide - est appliquée sur ledit revêtement.

2. Produit granulé ou comprimé selon la revendication 1, dans lequel la substance neutre est choisie dans le groupe constitué par les polymères hydrosolubles, les alcools supérieurs, les hydrates de carbone et les hydrocolloïdes et dans lequel ladite substance neutre est présente en une quantité allant d'environ 0,05 à environ 1,0 % en poids et, de préférence, d'environ 0,07 à environ 0,8 % en poids.

3. Produit granulé ou comprimé selon la revendication 1 ou la revendication 2, dans lequel un agent fixant l'humidité est fixé sur lesdits grains effervescents, cet agent fixant l'humidité étant choisi, de préférence, dans le groupe constitué par le carbonate de sodium anhydre et le sulfate de sodium anhydre et étant appliqué, de préférence, en une quantité allant d'environ 4 à environ 10 % en poids, par rapport au mélange total.

4. Produit granulé ou comprimé selon l'une quelconque des revendications précédentes, dans lequel on a appliqué sur les grains effervescents au moins un revêtement additionnel, comprenant une substance choisie dans le groupe constitué par les sels alcalins et/ou les sels alcalino-terreux d'au moins un acide organique alimentaire et solide servant de tampon et, à titre facultatif, une substance neutre additionnelle et dans lequel, de préférence, au moins un des revêtements contient un agent antimousse.

5. Produit effervescent granulé ou comprimé selon l'une quelconque des revendications précédentes, dans lequel le produit granulé ou ledit produit granulé pressé sous forme de comprimés comprend, en outre, au moins un agent antimousse présent lui-même sous forme d'un produit granulé séparé.

6. Produit granulé ou comprimé selon la revendication 4 ou la revendication 5, dans lequel l'agent antimousse est choisi dans le groupe constitué par la diméthicone et la siméthicone et est appliqué en une quantité d'environ 0,005 à environ 0,5 % en poids par rapport au mélange total ou d'environ 0,05 à environ 2,0 % en poids par rapport à la substance active du point de vue pharmaceutique.

7. Produit granulé ou comprimé selon l'une quelconque des revendications précédentes, ayant une capacité de fixation d'acides inférieure à 5 et, de préférence, inférieure à 3 méq., la détermination étant faite selon USP XXII.

8. Produit granulé ou comprimé selon l'une quelconque des revendications précédentes, qui, pour un poids total ne dépassant pas 2,5 et, de préférence 2,0 grammes, présente un temps de dissolution dans l'eau à la température ambiante inférieur à 180 et, de préférence, inférieur à 120 secondes.

9. Produit granulé ou comprimé selon l'une quelconque des revendications précédentes, qui comprend une substance active du point de vue pharmaceutique et hydrophobe, et dans lequel la substance hydrophobe est présente dans des granules distincts des composants effervescents, la substance hydrophobe de ces granules étant appliquée en revêtement ou fixée sur au moins une substance choisie dans le groupe constitué par des agents de suspension (choisis, de préférence, dans le groupe constitué par le produit Aerosil ® et le produit Avicel ®) et des substances neutres (choisies, de préférence, dans le groupe constitué par le mannitol et le sorbitol).

10. Produit granulé ou comprimé selon la revendication 9, dans lequel les granules contiennent également au moins un composant choisi dans le groupe constitué par des liants (de préférence la polyvinylpyrrolidone (PVP)), de petites quantités d'un tensioactif (choisi, de préférence, dans le groupe constitué par le dioctyl-sulfosuccinate de sodium et le lauryl-sulfate de sodium), et les carbonates et/ou les bicarbonates alcalins et/ou alcalino-terreux.

11. Produit granulé ou comprimé selon l'une quelconque des revendications précédentes contenant, par rapport au mélange total, d'environ 2 à environ 30 % en poids de cimétidine; d'environ 30 à environ 60 % en poids d'un acide organique alimentaire et solide; d'environ 12 à environ 40 % en poids d'au moins un carbonate ou un bicarbonate alcalin ou alcalinoterreux (dont d'environ 2 à environ 10 % en poids sont constitués par le carbonate de sodium utilisé comme agent fixant l'humidité); d'environ 1 à environ 4 % en poids d'un édulcorant; d'environ 0,01 à environ 30 % en poids d'une substance neutre (dont d'environ 0,01 à environ 0,05 % en poids servent pour le revêtement de la substance neutre), de préférence d'environ 3 à environ 20 % en poids de sorbitol et d'environ 2 à environ 10 % en poids de mannitol; d'environ 0,005 à environ 0,5 % en poids d'un agent antimousse et d'environ 0,1 à environ 3 % en poids d'un agent aromatisant.

12. Produit granulé ou comprimé selon l'une quelconque des revendications 1 à 10, contenant, par rapport au mélange total, les composants suivants : d'environ 0,4 à environ 4,5 % en poids de cisapride; d'environ 0,4 à environ 4,5 % en poids d'un agent de suspension; d'environ 0,1 à environ 1 % en poids d'un liant, de préférence la polyvinylpyrrolidone (PVP); d'environ 0,03 à environ 0,35 % en poids d'un tensioactif, de préférence le dioctyl-sulfosuccinate de sodium; d'environ 30 à environ 55 % en poids d'un acide organique alimentaire et solide, de préférence l'acide citrique; d'environ 12 à environ 40 % en poids d'au moins un carbonate ou un bicarbonate alcalin ou alcalino-terreux (dont d'environ 2 à environ 10 % en poids sont constitués par le carbonate de sodium utilisé comme agent fixant l'humidité); d'environ 0,3 à environ 2,5 % en poids d'un édulcorant; d'environ 0,02 à environ 55 % en poids d'une substance neutre (dont d'environ 0,02 à environ 0,1 % en poids servent pour le revêtement de la substance neutre), choisie, de préférence, dans le groupe constitué par la maltodextrine, le lactose et le mannitol; d'environ 0,005 à environ 0,05 % en poids d'agent antimousse, choisi, de préférence, dans le groupe constitué par la diméthicone et la siméthicone; et d'environ 0,2 à environ 5 % en poids d'un agent aromatisant.

13. Produit granulé ou comprimé selon l'une quelconque des revendications 1 à 10, contenant, par rapport au mélange total, les composants suivants :
- d'environ 0,1 à environ 0,5 % en poids de bêta-carotène (100 %);
- d'environ 0 à environ 2 % en poids d'acétate de tocophérol (100 %);
- d'environ 35 à environ 70 % en poids d'un acide organique alimentaire et solide, de préférence d'environ 0 à environ 10 % en poids d'acide ascorbique, d'environ 35 à environ 55 % en poids d'acide citrique et d'environ 0 à environ 5 % en poids d'acide malique;
- d'environ 11 à environ 38 % en poids d'au moins un carbonate ou un bicarbonate alcalin ou alcalino-terreux, de préférence d'environ 5 à environ 15 % en poids de carbonate de calcium et d'environ 5 à environ 20 % en poids de bicarbonate de sodium;
- d'environ 1 à environ 4 % en poids d'un édulcorant;
- d'environ 0,1 à environ 35,0 % en poids d'une substance neutre (dont d'environ 0,1 à environ 0,5 % en poids servent pour le revêtement de la substance neutre), de préférence d'environ 1 à environ 10 % en poids de sorbitol et d'environ 5 à environ 25 % en poids de mannitol; et
- d'environ 0,3 à environ 3 % en poids d'un agent aromatisant.

14. Produit granulé ou comprimé selon l'une quelconque des revendications 1 à 10, contenant, par rapport au mélange total, les composants suivants : d'environ 3 à environ 14 % en poids de chlorhydrate de ranitidine (75 - 300 mg par dose); d'environ 30 à environ 50 % en poids d'acide citrique; d'environ 0 à environ 20 % en poids de citrate monosodique; d'environ 10 à environ 30 % en poids de bicarbonate de sodium; d'environ 2 à environ 10 % en poids de carbonate de sodium; d'environ 1 à environ 3 % en poids d'un édulcorant; d'environ 0,05 à environ 0,2 % en poids de la substance neutre utilisée pour le premier revêtement ainsi que d'environ 0 à environ 15 % en poids de substances neutres additionnelles; d'environ 0 à environ 8 % en poids de granules d'un agent antimousse et d'environ 0,1 à environ 4 % en poids d'un agent aromatisant.

15. Comprimé effervescent contenant au moins une substance active sur le plan pharmaceutique et un système effervescent comprenant au moins un acide organique alimentaire et solide, au moins un carbonate ou un bicarbonate de métal alcalin en tant que composant générant du gaz et au moins un sel de métal alcalin de l'acide, dans lequel au moins deux couches sont appliquées aux cristaux porteurs constitués par au moins un premier acide, la première couche contenant au moins un autre acide organique alimentaire et solide ou un sel de métal alcalin de cet autre acide ou les deux, alors que la seconde couche contient au moins un sel de métal alcalin dudit premier acide, la première couche contenant en plus une substance neutre choisie dans le groupe constitué par les polymères hydrosolubles, les alcools supérieurs, les hydrates de carbone et les hydrocolloïdes.

16. Produit granulé ou comprimé avec un système effervescent selon l'une quelconque des revendications 1 - 15 et du cisapride en tant que substance active du point de vue pharmaceutique, qui, pour un poids total de moins de 2 grammes et, de préférence, de moins d'environ 1,6 grammes, a une capacité de fixation d'acides inférieure à 5 méq. et, de préférence, inférieure à 3 méq.

17. Produit granulé ou comprimé avec un système effervescent selon l'une quelconque des revendications 1 - 15 et de la cimétidine en tant que substance active du point de vue pharmaceutique, qui, pour un poids total de moins de 2,5 grammes et, de préférence, de moins d'environ 2,0 grammes, a une capacité de fixation d'acides inférieure à 5 méq. et, de préférence, inférieure à 3 méq.

18. Produit granulé ou comprimé avec un système effervescent selon l'une quelconque des revendications 1 - 15 et de la ranitidine en tant que substance active du point de vue pharmaceutique, qui, pour un poids total de moins de 2,6 grammes et, de préférence, de moins de 2,0 grammes, a une capacité de fixation d'acides inférieure à 3 méq. et, de préférence, inférieure à 2 méq.

19. Procédé de préparation d'un produit granulé ou un comprimé selon l'une quelconque des revendications précédentes, dans lequel des cristaux d'au moins un acide organique alimentaire et solide sont mouillés avec une solution aqueuse d'une substance neutre et ensuite, avant le séchage complet, un carbonate et/ou un bicarbonate alcalin et/ou alcalino-terreux, sous forme de poudre, est réparti de manière uniforme et fixé à la couche de surface humide par mélange, suite à quoi les grains effervescents ainsi préparés sont séchés et mélangés avec une substance active du point de vue pharmaceutique - qui est, de préférence, une substance sensible aux acides et qui est choisie, en particulier, dans le groupe constitué par les antagonistes des récepteurs H2, la cimétidine, la ranitidine, le cisapride et le bêta-carotène - et avec des adjuvants acceptables du point de vue pharmaceutique, puis éventuellement pressés en comprimés.

20. Procédé selon la revendication 19, dans lequel on applique sur les grains effervescents au moins un revêtement additionnel, en mouillant les grains avec une solution d'une substance tampon, de préférence choisie dans le groupe constitué par les carbonates alcalins, les bicarbonates alcalins, les carbonates alcalino-terreux, les bicarbonates alcalino-terreux, les sels alcalins d'au moins un acide organique alimentaire et solide et les sels alcalino-terreux d'au moins un acide organique alimentaire et solide.

21. Procédé selon la revendication 19 ou la revendication 20, dans lequel la solution comprend, en outre, une substance neutre choisie dans le groupe constitué par les polymères hydrosolubles, les alcools supérieurs, les hydrates de carbone et les hydrocolloïdes.

22. Procédé selon l'une quelconque des revendications 19 à 21, dans lequel, en plus du médicament, les grains effervescents sont également mélangés avec un produit granulé qui a été obtenu en appliquant un agent antimousse dans un solvant approprié sur la surface des particules de la substance neutre et en séchant le solvant.

23. Procédé selon l'une quelconque des revendications 19 à 22, dans lequel les grains effervescents séchés sont mouillés avec de l'éthanol qui contient, de préférence, un agent antimousse dissout, puis séchés à nouveau, en évaporant l'éthanol, pour enlever l'humidité résiduelle.

24. Procédé selon l'une quelconque des revendications 19 à 23, dans lequel, avant de mélanger la substance active du point de vue pharmaceutique au système effervescent, elle est appliquée en solution avec un agent liant et/ou un tensioactif, et répartie de manière uniforme sur les grains d'un agent de suspension et séchée.

25. Procédé selon l'une quelconque des revendications 19 à 24, dans lequel, avant de mélanger la substance active du point de vue pharmaceutique avec le système effervescent, elle est mélangée avec au moins une substance neutre, au moins un agent de suspension et au moins une substance choisie dans le groupe comprenant les carbonates alcalins, les bicarbonates alcalins, les carbonates alcalino-terreux, les bicarbonates alcalino-terreux, les sels alcalins d'au moins un acide organique alimentaire et solide et les sels alcalino-terreux d'au moins un acide organique alimentaire et solide, suite à quoi une solution d'au moins un agent liant et/ou d'un tensioactif est appliquée et répartie sur les grains du mélange, qui sont alors séchés.

26. Procédé de fabrication de granules effervescents à partir d'un mélange pulvérulent ou d'un mélange granulé d'un acide organique alimentaire et solide et d'un carbonate et/ou d'un bicarbonate d'un métal alcalin ou alcalino-terreux sous vide, dans lequel, pour la passivation de la surface d'au moins un des composants pour l'amener dans un état de haute inertie à la réaction, on ajoute au mélange chauffé durant le traitement sous vide, une quantité mesurée d'un solvant polaire, la différence de pression provoquée par la formation de gaz carbonique produit par l'addition du solvant durant la réaction étant choisie pour atteindre au maximum 1000 mbars, le volume et la masse du gaz carbonique libéré étant déterminés à partir de cette différence de pression, et on répète le traitement thermique, après un séchage rapide du mélange, autant de fois que nécessaire pour obtenir une passivation de la surface, comme indiqué par un ralentissement évident de la réaction et par une formation diminuée de gaz, une substance neutre choisie dans le groupe constitué par les polymères hydrosolubles, les alcools supérieurs, les hydrates de carbone et les hydrocolloïdes étant dissoute dans ledit solvant polaire.

27. Procédé de préparation d'un matériau granulé effervescent contenant au moins un acide organique alimentaire cristallin et solide et au moins un carbonate d'un métal alcalin ou d'un métal alcalino-terreux produisant du CO₂ par réaction avec ledit acide organique en solution aqueuse, qui comprend les opérations consistant à :
- provoquer une réaction préliminaire d'une portion dudit acide organique et dudit carbonate en solution dans de l'eau et/ou un alcool pour former un produit de réaction préliminaire,
- ajouter ledit produit de réaction préliminaire à une portion additionnelle dudit acide organique sous forme cristalline et procéder à un mélange poussé pour former un premier revêtement par réaction avec lesdits cristaux d'acide organique et libération d'eau de cristallisation résultante,
- appliquer au moins un revêtement additionnel comprenant ledit carbonate sur les cristaux d'acide organique avec ledit premier revêtement adhérant à ceux-ci; et
- terminer la réaction après que le dernier revêtement a été appliqué, par un séchage, une substance neutre choisie dans le groupe constitué par les polymères hydrosolubles, les alcools supérieurs, les hydrates de carbone et les hydrocolloïdes étant ajoutée audit produit de réaction préliminaire.
